# EUROPÄISCHE PATENTSCHRIFT

(11) **EP 1 374 780 B1**
(45) Veröffentlichungstag und Bekanntmachung des Hinweises auf die Patenterteilung: **31.01.2007**
(21) Anmeldenummer: 03010746.0
(22) Anmeldetag: 14.05.2003
(51) Int. Cl.: A61B 17/17, A61B 17/72

(54) **Implantationssystem und Zielgerät dafür**
Implantation system and target device therefor
Système d'implantation et appareil de visée associé

(30) Priorität: 20.06.2002 DE 10227379
(43) Veröffentlichungstag der Anmeldung: 02.01.2004
(73) Patentinhaber: Stryker Trauma GmbH, 24232 Schönkirchen (DE)
(72) Erfinder: Anastopoulos, George, Dr., 16341 Athen (GR); Robioneck, Paul Bernd, 24211 Preetz (DE); Lutz, Christian, 4500 Solothurn (CH)
(74) Vertreter: Kopf, Korbinian Paul

(56) Entgegenhaltungen:
- WO-A-01/60272
- WO-A-96/03085
- DE-A1- 4 240 277
- DE-U1- 20 020 989
- DE-U1- 29 815 700
- US-A- 5 429 640

## Beschreibung

Die Erfindung bezieht sich auf ein Implantationssystem nach dem Patentanspruch 1.

Verriegelungsnägel zur Versorgung von Frakturen von Röhrenknochen sind weit verbreitet. Ihre Anwendung ist etwa in "The Journal of Trauma" von 1993 Vol. 35, Nr. 5, Seiten 772 bis 775 beschrieben. Typisch für derartige Verriegelungsnägel ist die Anordnung von zwei Querbohrungen an einem Ende (z.B. distalem Ende) und mindestens einer Querbohrung an dem anderen Ende (z.B. proximalen Ende). Durch die Querbohrungen werden Knochenschrauben hindurchgeführt, die an gegenüberliegenden Seiten in die Kortikalis eingeschraubt werden. Dadurch ist der Verriegelungsnagel axial und gegen Drehung gesichert.

Beim Einsatz derartiger Verriegelungsnägel muss die Lage der Querbohrungen oder Verriegelungslöcher im Verriegelungsnagel identifiziert werden, damit die Kortikalis von außen an der richtigen Stelle gebohrt wird. Hierfür ist eine Reihe von Zielgeräten bekannt geworden, die üblicherweise mit Röntgenstrahlen arbeiten, um die Lage der Querbohrungen relativ zum Zielgerät zu ermitteln. Ist dies der Fall, wird mit Hilfe des Zielgerätes und einer sogenannten Bohr- oder Zielhülse der Knochen an der richtigen Stelle gebohrt, damit die Knochenschrauben eingedreht werden können. Bekannte Zielgeräte werden üblicherweise mit dem einen Ende (z.B. proximales Ende) des Nagels verbunden. Auf diese Weise ist die Zuordnung der Verriegelungslöcher zu Zielbohrungen im Zielgerät annähernd vorgegeben. Allerdings ist zu berücksichtigen, dass durch die Krümmung des Knochens und durch mögliche Torsion des Nagels beim Eintreiben die vermutete Lage der Verriegelungslöcher nicht mit der tatsächlichen übereinstimmt.

Es ist daher bisher nicht möglich gewesen, eine genaue Lageidentifizierung der Verriegelungslöcher auf nur mechanischem Wege vorzunehmen.

Aus WO01/60272 A1 ist ein Verriegelungsnagel bekannt geworden, bei dem lediglich im distalen Abschnitt im Bereich der Verriegelungslöcher eine achsparallele Nut im Nagelschaft eingeformt ist. Die Lenkachse der Nut schneidet die Achse der Bohrung annähernd senkrecht. In der erwähnten Druckschrift ist auch ein Zielgerät beschrieben, dessen Zielarm zwei Zielbohrungen aufweist. Die Zielbohrungen sind in einem Abschnitt des Zielarms des Zielgeräts geformt, der elastisch nachgebend ist. Mit Hilfe eines dünnen stabartigen Fühlers wird mit Hilfe der Verriegelungslöcher und der Nut dazwischen die tatsächliche Lage des Nagels im Knochen ermittelt. Durch Drehen des Nagels und axiales Verschieben kann festgestellt werden, wann der Fühler sich im Bereich eines Verriegelungsloches befindet.

Aus WO 96/03085 ist bekannt einen einen Verriegelungsnagel aufnehmenden Adapter in ein Verbindungsteil einzusetzen, das axial verschiebbar und schwenkbar an einem starren Zielarm eines Zielgeräts gelagert ist.

Es hat sich herausgestellt, dass dieses rein mechanische Verfahren für den Chirurgen noch Probleme bereitet, da er durch Betätigung des Fühlers die tatsächliche Lage des Nagels nicht immer genau genug ermitteln kann.

Der Erfindung liegt daher die Aufgabe zugrunde, ein Implantationssystem bzw. ein Zielgerät zu schaffen, mit dem auf einfache Weise die Lage der Verriegelungslöcher festgestellt und die Bohrungen im Knochen an der richtigen Stelle gesetzt werden können.

Diese Aufgabe wird durch die Merkmale des Patentanspruchs 1 gelöst.

Der Verriegelungsnagel bei dem erfindungsgemäßen Implantationssystem weist Mittel auf, mit deren Hilfe die Lage der Verriegelungslöcher aufgefunden werden kann. Ein Mittel besteht zum Beispiel darin, eine achsparallele Nut zwischen den Verriegelungslöchern anzuordnen. Eine andere Möglichkeit besteht darin, im Bereich der Verriegelungslöcher Umfangsnuten vorzusehen oder alternativ geeignete Anschläge, die mit Hilfe eines Fühlers erfasst werden können.

Die Besonderheit bei dem erfindungsgemäßen System besteht jedoch in dem Zielgerät dahingehend, dass der Zielarm starr ausgebildet ist und einen Zielabschnitt aufweist, in dem drei Zielbohrungen vorgesehen sind. Die Achsen der Zielbohrungen liegen in einer Ebene, die in der Längsachse des Zielarms oder parallel dazu verläuft. Der Abstand der äußeren Zielbohrungen entspricht dem Abstand der Verriegelungslöcher. Die mittlere Zielbohrung kann irgendwo zwischen den äußeren Zielbohrungen liegen, zum Beispiel auf der halben Strecke des Abstands. Der Verriegelungsnagel ist mit einem Nageladapter lösbar verbindbar, beispielsweise durch eine geeignete Schraubenverbindung. der Nageladapter ist lösbar mit einem Verbindungsteil verbindbar, das seinerseits lösbar mit dem starren Zielarm verbindbar ist. Die lösbare Verbindung des Nageladapters mit dem Zielarm ermöglicht die Verwendung des erfindungsgemäßen Zielgerätes für eine Vielzahl unterschiedlicher Verriegelungsnägel, ohne dass für jeden Typ Verriegelungsnagel ein separates Zielgerät erforderlich ist. Durch die Verstellung zwischen Adapter einerseits und Zielarm andererseits kann vor dem Implantieren der Nagel relativ zum Zielarm justiert werden. Dies geschieht auf einfache Weise dadurch, dass z.B. mit Hilfe eines Kalibrierstifts die äußeren Zielbohrungen des Zielabschnitts des Zielarms mit den Verriegelungslöchern des Nagels ausgerichtet werden. Anschließend kann der Nagel implantiert werden, wobei der Nageladapter am Nagel befestigt bleiben kann. Der Verriegelungsnagel wird nun entweder weiter eingeschlagen als seine endgültige Lage erfordert oder auch weniger weit als die erwünschte Endlage. Anschließend wird das Zielgerät wieder zusammengebaut und über die mittlere Zielbohrung die zugewandte Kortikalis aufgebohrt. Danach wird das Zielgerät wieder vom Nageladapter gelöst und ein stabartiger Fühler durch die Knochenbohrung hindurchgesteckt. Der Fühler wird gegen die geschlossene Wand des Verriegelungsnagels stoßen und im günstigsten Fall z.B. in die Nut hinein, welche die Verriegelungslöcher verbindet. Durch Drehen des Nagels kann z.B. die Nut gefunden und festgestellt werden, dass es sich tatsächlich um diese handelt. Anschließend wird der Nagel herausgezogen oder weiter hineingetrieben, bis der Fühler mit dem zugeordneten Verriegelungsloch zusammenwirkt. Anschließend wird das Zielgerät wieder montiert, und eine der äußeren Zielbohrungen wird mit dem gefundenen Verrriegelungsloch ausgerichtet. Hierzu sind verschiedene Möglichkeiten denkbar. Vorzugsweise wird eine Fixierhülse verwendet, die einen vorderen Abschnitt aufweist, dessen Durchmesser dem Durchmesser des Verriegelungsloches und einen anderen Abschnitt, dessen Durchmesser der Zielbohrung entspricht. Der Innendurchmesser der Fixierhülse ist so bemessen, dass er auf den stabförmigen Fühler, der sich noch im Verriegelungsloch und der Knochenbohrung befindet, geschoben werden kann. Auf diese Weise lässt sich eine ziemlich präzise Ausrichtung einer Zielbohrung mit einem Verriegelungsloch erreichen, indem de Zielarm wieder montiert und seine eine Zielbohrung auf die Fixierhülse geschoben wird. Ist dies geschehen, dann ist auch die zweite Zielbohrung im Zielarm mit dem zweiten Verriegelungsloch ausgerichtet, und es kann in bekannter Weise die Kortikalis über dem zweiten Verriegelungsloch aufgebohrt werden, damit anschließend das Setzen der Verriegelungsschraube erfolgen kann. Ist die erste Verriegelung durchgeführt worden, kann auch die zweite Verriegelung erfolgen, indem über die Zielbohrung, über die zunächst die Ausrichtung erfolgte, die Kortikalis ebenfalls in bekannter Weise aufgebohrt wird, um die zweite Verriegelungsschraube zu setzen.

Wie schon erwähnt, weist der Verriegelungsnagel im Bereich der Verriegelungslöcher Mittel auf, mit denen das Erfühlen des Nagels und das Ertasten eines Verriegelungsloches erleichtert wird.

Es ist jedoch theoretisch auch denkbar, ohne derartige Mittel auszukommen, dies allerdings nur, wenn der Verriegelungsnagel beim Eintreiben nicht um den Durchmesser des Verriegelungsloches tordiert wurde. Ist dies der Fall, dann weiß der Chirurg nicht, wieviel der Nagel herausgezogen oder eingeschlagen werden muss, bis die axiale Lage des Verriegelungsloches erreicht ist.

Für das Kalibrieren ist erfindungsgemäß vorgesehen, daß der Zielabschnitt um eine Achse senkrecht zur Längsachse des Zielarms verschwenkbar gelagert ist und erste Feststellmittel aufweist zur Festlegung des Zielabschnitts in einer vorgegebenen Schwenkposition. Die Schwenkachse liegt auf der Achse der Zielbohrung, die dem anderen Ende des Nagels am nächsten liegt.

Nach einer Ausgestaltung der Erfindung weist der Zielabschnitt im Bereich der Zielbohrungen einen achsparallelen Schlitz auf, der sich senkrecht zur Achse der Zielbohrungen erstreckt und den Zielabschnitt in einen schmaleren und einen breiteren Abschnitt unterteilt. Auf diese Weise wird beim Einsetzen von Kalibrierstift, Bohrhülse oder dergleichen eine klemmende Wirkung erhalten, sodass diese Teile nicht ungewollt verrutschen oder herausfallen.

Die Kalibrierung wird ferner nach einer Ausgestaltung der Erfindung dadurch erleichtert, wenn der Nageladapter um seine Längsachse schwenkbar im Verbindungsteil gelagert ist und zweite Feststellmittel vorgesehen sind, um die relative Schwenklage festzulegen. Nach einer weiteren Ausgestaltung der Erfindung sind zwischen dem Verbindungsteil und dem Nageladapter Einstellmittel vorgesehen zur Einstellung der relativen Schwenklage. Die Einstellmittel können z.B. eine Einstellschraube oder dergleichen enthalten.

Der starre Zielarm des erfindungsgemäßen Zielgerätes ist vorzugsweise als längliche Stange mit unrundem Querschnitt ausgebildet, die in eine komplementäre Öffnung des Verbindungsteils eingeführt ist, wobei eine Wand der Öffnung von einem Klemmelement gebildet ist, das von einer Klemmschraube betätigbar ist. Auf diese Weise kann das Verbindungselement stufenlos auf dem Zielarm axial verstellt werden, wobei die Drehlage unverändert bleibt. Weitere Ausgestaltungen der Erfindung sind in weiteren Unteransprüchen angegeben.

Die Erfindung wird nachfolgend anhand von Zeichnungen näher erläutert.
- Fig. 1: zeigt ein Zielgerät nach der Erfindung in Seitenansicht.
- Fig. 2: zeigt eine um 90° verdrehte Seitenansicht des Zielgeräts nach Fig. 1.
- Fig. 3: zeigt das Zielgerät nach den Figuren 1 und 2 in perspektivischer Darstellung.
- Fig. 4: zeigt das Ende des Zielarms des Zielgeräts nach den Figuren 1 bis 3.
- Fig. 5: zeigt eine Endansicht eines Zielabschnitts des Zielgeräts nach den Figuren 1 bis 3.
- Fig. 6: zeigt einen Schnitt durch die Darstellung nach Fig. 5 entlang der Linie 6-6.
- Fig. 7: zeigt eine um 90° verdrehte Endansicht des Zielabschnitts nach Fig. 5.
- Fig. 8: zeigt perspektivisch das Verbindungs- und Kalibrierteil des Zielgeräts nach den Figuren 1 bis 3.
- Fig. 9: zeigt das Verbindungsteil der Anordnung nach Fig. 8.
- Fig. 10: zeigt einen Schnitt durch das Verbindungsteil nach Fig. 9.
- Fig. 11: zeigt eine Seitenansicht des Kalibrier- und Verbindungsteils nach Fig. 8.
- Fig. 12: zeigt eine Einzelheit der Anordnung nach Fig. 11.
- Fig. 13: zeigt die Seitenansicht eines Kalibrierstifts.
- Fig. 14: zeigt die Seitenansicht eines Fühlers.
- Fig. 15: zeigt die um 90° verdrehte Seitenansicht des Fühlers nach Fig. 14.
- Fig. 16: zeigt die Seitenansicht einer Fixierhülse.
- Fig. 17: zeigt die Seitenansicht eines Verriegelungsnagels sowie eines daran befestigten Nageladapters.
- Fig. 18: zeigt das Ende eines Nagels in einer weiteren Ausführungsform.
- Fig. 19: zeigt eine weitere Ausführungsform eines Endes des Verriegelungsnagels.
- Fig. 20: zeigt die Seitenansicht eines Nageladapters für das System nach den vorangehenden Figuren.

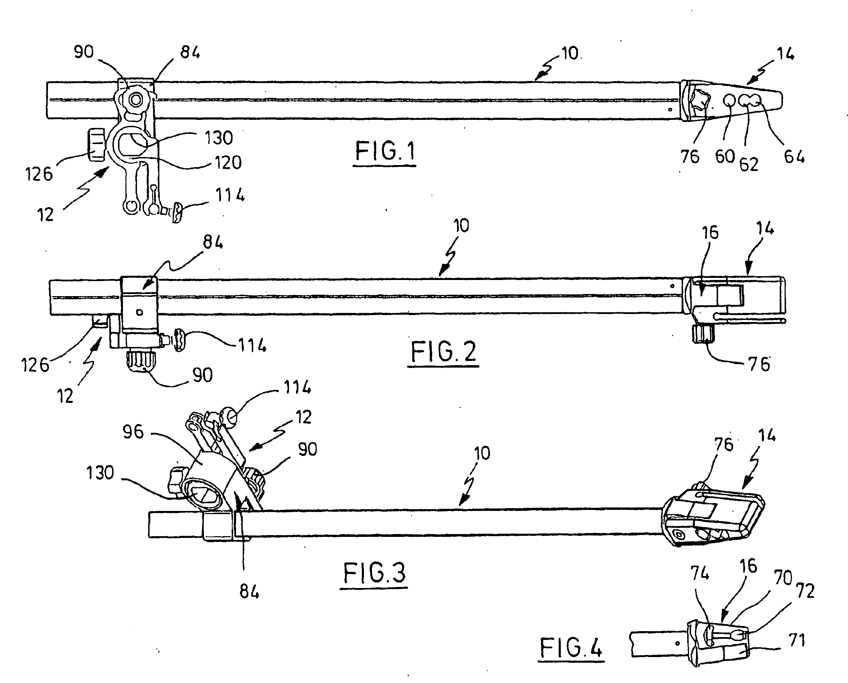

In den Figuren 1 bis 3 ist ein Zielarm 10 zu erkennen, der quadratischen Querschnitt aufweist. Am linken Ende des Zielarms 10 sitzt ein Kalibrier- und Verbindungsteil 12, und am rechten Ende ist ein Zielabschnitt 14 zu erkennen, der auf einem geeigneten Lagerabschnitt 16 am rechten Ende des Zielarms 10 schwenkbar gelagert ist.

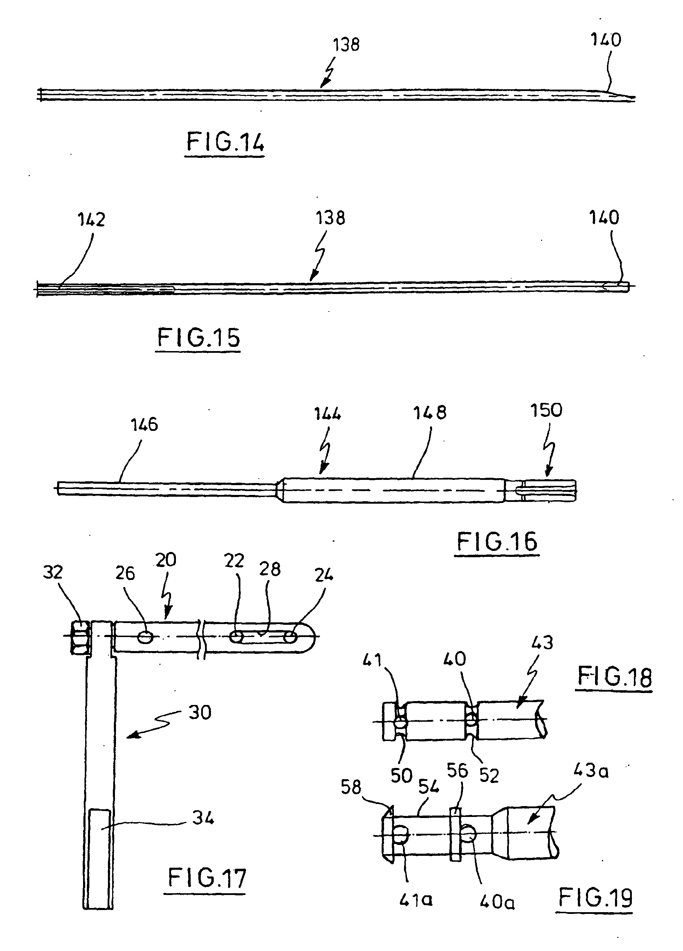

Verriegelungsnägel, für welche das Zielgerät nach den Figuren 1 bis 3 verwendbar ist, sind andeutungsweise in den Figuren 17 bis 19 dargestellt. In Fig. 17 ist ein Verriegelungsnagel 20 zu erkennen, beispielsweise ein Femurnagel. Er weist am distalen Ende zwei axial beabstandete Verriegelungslöcher 22, 24 auf und am proximalen Ende ein Verriegelungsloch 26. Zwischen den Verriegelungslöchern 22, 24 erstreckt sich eine achsparallele Nut 28 von z.B. U- oder V-förmigem Querschnitt. Der Durchmesser der Verriegelungslöcher 22 bis 26 ist gleich. Im gezeigten Beispiel ist das proximale Ende des Verriegelungsnagels 20 mit einem länglichen Nageladapter verbunden, und zwar mit Hilfe einer Schraube 32, die in das proximale Ende des Nagels 20 einschraubbar ist. Der Nageladapter weist am anderen Ende gegenüberliegende Abflachungen 34 auf.

In Fig. 18 ist das Ende eines Verriegelungsnagels 43 gezeigt mit Verriegelungslöchern 40, 41 und Umfangsnuten 50, 52 im Bereich der Verriegelungslöcher 40, 41. In Fig. 19 sind Verriegelungslöcher 41, 40a gezeigt, die in einem im Durchmesser verkleinerten distalen Endabschnitt des Verriegelungsnagels 43a geformt sind. Jeweils links von den Verriegelungslöchern 40a, 41a ist ein im Durchmesser radialer Bund 56, 58 geformt, wobei der Bund 58 zum linken Ende hin angeschrägt ist. Auf die Funktion der gezeigten Nagelausbildungen wird weiter unten noch eingegangen.

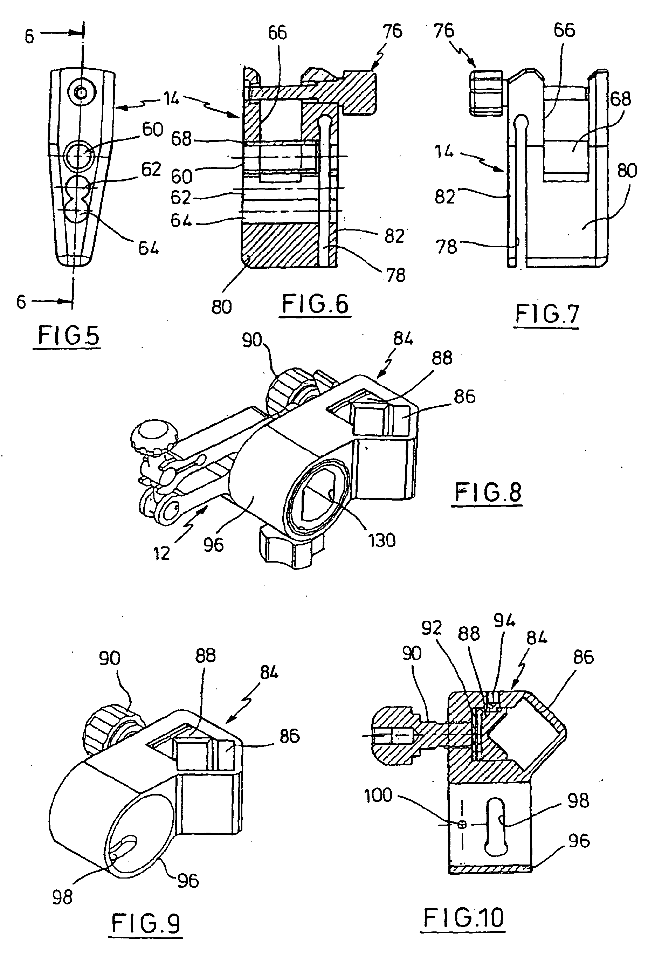

Wie man insbesondere aus den Figuren 5 bis 7 entnimmt, weist der Zielabschnitt 14 drei Zielbohrungen 60, 62, 64 auf, deren Achsen in einer Ebene liegen, die durch die Längsachse des Zielarms 10 geht oder parallel zu dieser verläuft. Der Achsabstand der äußeren Zielbohrungen 60, 64 entspricht dem Abstand der Verriegelungslöcher 22, 24, bzw. 40, 41, bzw. 40a, 41a. Wie aus den Figuren 6 und 7 hervorgeht, weist der Zielabschnitt 14 einen ersten relativ breiten Schlitz 66 auf, dessen Breite geringfügig größer ist als die Breite des Lagerabschnitts 16. Wie aus Fig. 4 hervorgeht, ist der Lagerabschnitt gabelartig mit Armen 70, 71 ausgebildet, die zwischen sich eine Erweiterung 72 aufweisen nahe den Enden der Arme 70, 71 und einen bogenförmigen quer verlaufenden Schlitz 74 am Ende der Arme 70, 71. Aus den Figuren 6 und 7 geht hervor, dass der Schlitz 66 vom Schaft einer Feststellschraube 76 durchsetzt ist, wobei der Durchmesser des Schaftes etwas geringer ist als die Breite des bogenförmigen Schlitzes 74 des Lagerabschnitts 16. Am inneren Ende ist der Schlitz 66 von einer Lagerhülse 68 durchsetzt. Wird der Lagerabschnitt 16 in den Schlitz eingeführt, gelangt der Schaft der Feststellschraube 76 in den bogenförmigen Schlitz 74 und die Hülse 68 in die Erweiterung 72. Somit ist der Zielabschnitt 14 um die Achse der Hülse 68 schwenkbar im Lagerabschnitt 16 gelagert. Die relative Schwenklage wird durch die Feststellschraube 76 festgelegt.

Durch einen relativ schmaleren Schlitz 78, der vom freien Ende des Zielabschnitts 14 her eingebracht ist, wird der Zielabschnitt 14 in einen relativ breiten Abschnitt 80, der den Schlitz 66 aufweist und einen relativ schmaleren Abschnitt 82 unterteilt. Letzterer ist relativ zum ersteren begrenzt biegbar und weist Bohrungen auf, die mit den Zielbohrungen 60 bis 64 ausgerichtet sind.

In den Figuren 8 bis 10 ist ein Verbindungsteil 84 dargestellt, mit dessen Hilfe der Nageladapter zum Beispiel nach Fig. 17 am Zielarm 10 verstellbar und lösbar gehalten werden kann. Das Verbindungsteil 84 weist eine quadratische Öffnung 86 auf von einem Querschnitt, der dem Querschnitt des Zielarms 10 entspricht. Ein Klemmabschnitt 88 ist verschiebbar im Verbindungsteil 84 gelagert und bildet einen Eckabschnitt der Öffnung 86. Der Abschnitt 88 wird durch eine Klemmschraube 90 betätigt, die auf einen relativ dünnen Abschnitt 92 des Abschnitts 88 wirkt, der durch einen Schlitz vom übrigen Abschnitt 88 getrennt ist, um eine nachgebende Wirkung zu erhalten. Ein Stift 94, der in einer Nut des Abschnitts 88 sitzt, begrenzt die Verstellung des Abschnitts 88.

Das Verbindungsteil 84 weist außerdem eine Lagerhülse 96 auf, deren Achse senkrecht zur Achse der Aufnahmeöffnung 86 verläuft. In der Lagerhülse 96 ist ein Schlitz 98 eingeformt, der eine gewisse Erstreckung in Umfangsrichtung hat. Ferner ist ein Stift 100 im Presssitz eingelassen, der ein wenig über das Innere der Lagerhülse 96 hervorsteht.

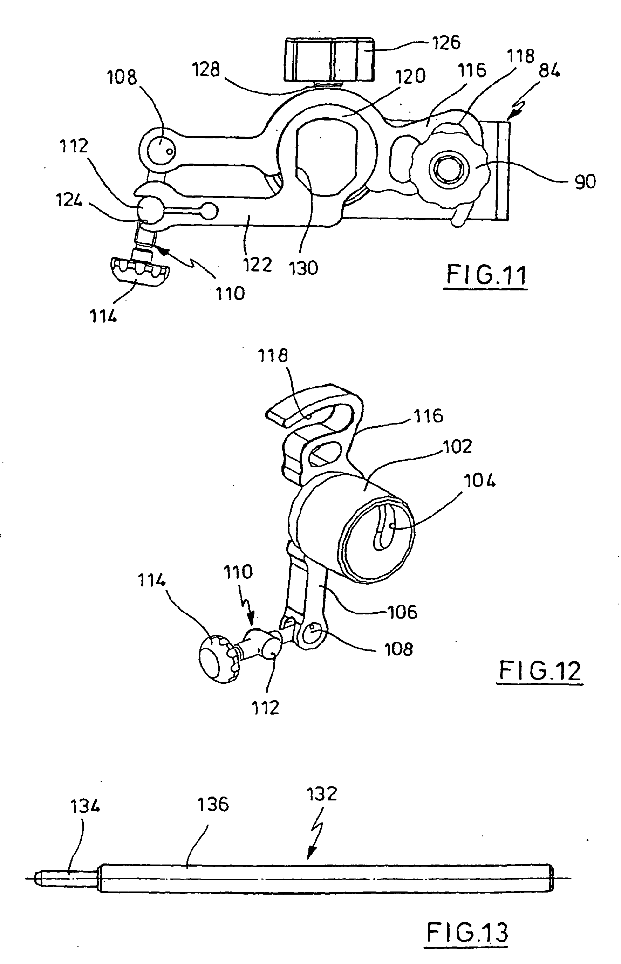

In Fig. 11 ist die andere Seite des Kalibrier- und Verbindungsteils nach Fig. 8 in Seitenansicht dargestellt. In Fig. 12 ist ein Hülsenabschnitt 102 zu erkennen, der einen Schlitz 104 aufweist, der sich teilweise in Umfangsrichtung erstreckt. Der Hülsenabschnitt 102 ist so bemessen, dass er in die Lagerhülse 96 eingesetzt werden kann, wobei die Schlitze 98 und 104 zumindest teilweise zur Deckung gelangen können. An einem Ende des Hülsenabschnitts ist ein Arm 106 angebracht, der bei 108 eine Einstellschraube 110 schwenkbar lagert, die mit einem zylindrischen Knebel 112 versehen ist, der ein Innengewinde aufweist. Die Betätigung der Schraube 110 erfolgt über einen Drehknopf 114. Dem Arm 106 gegenüberliegend ist am gleichen Ende am Hülsenabschnitt 102 ein Arm 116 angeformt, der einen gebogenen zur einen Seite offenen Schlitz 118 aufweist. Innerhalb des Hülsenabschnitts 102 befindet sich ein weiterer Hülsenabschnitt 120, an den ein Arm 122 angeformt ist. Am freien Ende des Arms 122 ist eine kreisförmige Ausnehmung 124 geformt, welche den runden Knebel 112 aufnimmt. Bei einer Verdrehung des Knopfes 114 findet mithin eine Relatiwerschwenkung zwischen den Armen 108 und 122 statt. Mit einem Knopf 126 wird eine Feststellschraube 128 betätigt, die in eine nicht gezeigte Gewindebohrung des Hülsenabschnitts 120 eingreift. Der Schaft der Schraube 128 erstreckt sich dabei durch die Schlitze 98 und 104 von Lagerhülse 96 und Hülsenabschnitt 102. Der Hülsenabschnitt 120 weist eine unrunde Öffnung 130 auf für die Aufnahme des Endabschnitts des Nageladapters 30. Mit Hilfe der Feststellschraube 128 kann die axiale Lage des Nageladapters in der Öffnung 130 festgelegt werden. Durch Verschwenkung des Arms 122 relativ zum Verbindungsteil 84 kann der Nageladapter um seine Achse gedreht werden und dabei eine Verschwenkung des Nagels 20 vornehmen. Die relative Schwenklage des Hülsenabschnitts 102 bzw. der Arme 106 und 116 bezüglich des Verbindungsteils 84 wird durch die Feststellschraube 90 bewirkt, deren Schaft sich durch den gebogenen Schlitz 118 erstreckt.

In Fig. 13 ist ein Kalibrierstift 132 gezeigt mit einem linken Abschnitt 134 von kleinerem Durchmesser und einem relativ langen Abschnitt 136 von größerem Durchmesser. Der Durchmesser des Abschnitts 134 entspricht dem Durchmesser der Verriegelungsbohrungen 26, 24 bzw. 40, 41 bzw. 40a, 41a. Der Durchmesser des Abschnitts 136 entspricht dem Durchmesser der Zielbohrungen 60, 62 bzw. 64.

In den Figuren 14 und 15 ist ein Fühler 138 gezeigt, der über seine Länge einen kreisförmigen Querschnitt hat von relativ geringem Durchmesser hat, der deutlich kleiner ist als der Durchmesser der Verriegelungslöcher. An einem Ende ist der Fühler 138 angeschrägt, wie bei 140 gezeigt zur Bildung einer Art Schneide. Am anderen Ende weist der Fühlerstift 138 gegenüberliegend Abflachungen 142 auf zwecks Einspannung in einen Griffabschnitt (nicht gezeigt).

In Fig. 16 ist eine Fixierhülse 144 dargestellt mit einem Abschnitt 146, dessen Außendurchmesser dem Durchmesser der Verriegelungslöcher 22, 24 bzw. 41, 40, 41a, 40a entspricht. Ein Abschnitt 148 hat einen größeren Durchmesser, der dem Durchmesser der Zielbohrungen 60 bis 64 entspricht. Am rechten Ende ist die Zielhülse 144 mit einem Einspannabschnitt 150 versehen zwecks lösbarer Verbindung mit einer Handhabe. Die Zielhülse 144 weist eine durchgehende axiale Bohrung auf von einem Durchmesser derart, dass der Fühlerstift 138 annähernd passend hindurchgesteckt werden kann.

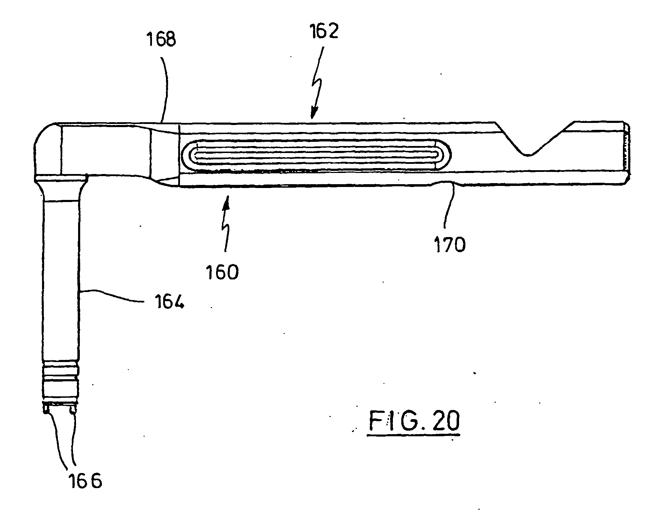

Der in Fig. 20 gezeigte Nageladapter 160 weist einen Schaft 162 auf, dessen Querschnitt komplementär ist zum Querschnitt der Öffnung 130 im Verbindungsteil 84 (siehe z.B. Fig. 8). Am linken Ende des Schaftes 162 ist ein Adapterabschnitt 164 mit Hilfe einer nicht gezeigten Schraubverbindung angebracht. Er weist am freien Ende zwei Vorsprünge 166 auf, die in entsprechende Ausnehmungen des Nagels (hier nicht gezeigt) eingreifen, um seine Drehlage vorzugeben. Der Nagel wird dann mit Hilfe einer Schraubverbindung, welche sich durch den hohlen Abschnitt 164 hindurcherstreckt, am Adapter 160 befestigt.

Bei 168 bzw. 170 sind Bohrungen durch den Schaft 182 angedeutet, die auch als Zielbohrungen verwendet werden können.

Das beschriebene Implantationssystem wird wie folgt angewendet. Zunächst erfolgt eine Kalibrierung. Der zu implantierende Nagel, beispielsweise der Nagel 20 nach Fig. 17 wird mit dem Nageladapter 30 verbunden. Der Nageladapter 30 wird in die Öffnung 130 des Verbindungsteils eingesetzt und mit Hilfe der Klemmschraube 126 festgelegt. Durch axiales Verstellen des Verbindungsteils 84 auf dem Zielarm 10 und ggf. durch Verschwenkung des Nagels, indem die Einstellschraube 114 betätigt wird, werden die Verriegelungslöcher 22, 24 zu den Zielbohrungen 60, 64 ausgerichtet. Zunächst erfolgt eine Ausrichtung bezüglich der Zielbohrung 60, indem ein Kalibrierstift gemäß Fig. 13 hindurchgeführt wird. Wahlweise kann hierfür auch eine Bohrerführungshülse und ein Bohrer eingesetzt werden. Dabei gelangt der Abschnitt 134 des Kalibrierstifts 132 in das Verriegelungsloch 22. Anschließend wird der Zielabschnitt 14 so weit verschwenkt, bis auch die Zielbohrung 64, 84 zum Verriegelungsloch 24 ausgerichtet ist, was mit Hilfe des Kalibrierstifts festgestellt werden kann. Danach erfolgt auch eine Festsetzung des Zielabschnitts 14 in der eingenommenen Schwenkstellung mit Hilfe der Feststellschraube 76. Die Lage des Nagels war zuvor bereits durch die Klemmschraube 126, die Feststellschraube 90 und die Einstellschraube 114 eingestellt. Anschließend wird der Nageladapter 30 durch Lösen der Klemmschraube 126 entfernt, und der Verriegelungsnagel 20 wird in üblicher Weise in den Knochen eingetrieben, beispielsweise von proximal in den Femurkanal. Der Nageladapter 30 bleibt am Verriegelungsnagel 20 befestigt. Der Verriegelungsnagel 20 wird um eine gewisse Strecke weiter eingetrieben als die gewünschte Endlage. Dieses Übermaß entspricht einer Strecke, die kleiner ist als der Abstand der Verriegelungslöcher 22, 24. Nachdem der Nagel eingetrieben worden ist, erfolgt eine Befestigung des Zielarms 10 am Nageladapter 30 in der bereits beschriebenen Art und Weise. Nunmehr wird durch die mittlere Zielbohrung 62 mit Hilfe einer geeigneten Bohrhülse und ggf. Gewebeschutzhülse ein Loch in die zugewandte Kortikalis des Knochens gebohrt. Hierbei soll vermieden werden, dass der Bohrer mit dem Nagel 20 in Kontakt kommt. Ist dies geschehen, wird anschließend das Zielgerät wieder vom Nageladapter entfernt, und es wird mit Hilfe des Fühlers 138, der durch die Knochenbohrung hindurchgesteckt wird, versucht, die Nut 28 zu erfühlen. Ist dies nicht der Fall, kann durch leichtes Drehen des Nagels 20 im Knochenkanal die Nut 28 ertastet werden. Sobald dies der Fall ist, wird der Nagel 20 etwas herausgezogen, bis der Fühler 22 das Verriegelungsloch 24 ertastet. Ist dies der Fall, wird über den Fühler 138 die Fixierhülse 144 geschoben. Der Abschnitt 146 gelangt dabei durch die Knochenbohrung in das Verriegelungsloch 24. Mit derart einsitzender Verriegelungshülse 144 wird anschließend das Zielgerät erneut montiert, wobei die Zielbohrung 64 zur Fixierhülse 144 ausgerichtet wird. Ist eine Befestigung des Zielgeräts erfolgt, ist automatisch die innere Zielbohrung 60 zum inneren Verriegelungsloch 22 ausgerichtet. Jetzt kann mit Hilfe der üblichen Methoden der Knochen auf gegenüberliegenden Seiten des Verriegelungsloches 22 aufgebohrt werden und eine Verriegelungsschraube eingeführt werden, um den Nagel im Knochen festzulegen. Ist dies geschehen, kann auch durch Entfernen der Fixierhülse 144 und mit Hilfe der üblichen Methoden die Kortikalis auch auf der anderen Seite aufgebohrt werden, um auch in diesen Bereich eine Verriegelungsschraube einzubringen.

Sobald die distale Verriegelung beendet ist, kann auch eine Verriegelung im Bereich des proximalen Verriegelungsloches 26 in bekannter Art und Weise erfolgen.

## Patentansprüche

1. Implantationssystem aus mindestens einem Verriegelungsnagel (20, 43, 43a) und einem Zielgerät, wobei der Verriegelungsnagel (20, 43, 43a) an einem Ende zur lösbaren Befestigung am Zielgerät ausgebildet ist und am anderen Ende zwei Verriegelungslöcher (22, 24; 40, 41; 40a, 41a) aufweist und der Verriegelungsnagel (20, 43, 43a) eine äußere, die Verriegelungslöcher verbindende achsparallele Nut (28) oder in Umfangsrichtung verlaufende Vertiefungen (50, 52) oder Erhebungen (56, 58) im Bereich der Verriegelungslöcher aufweist und das Zielgerät einen Zielarm (10) aufweist mit einem Zielabschnitt (14) am freien Ende, der in einer achsparallelen Ebene angeordnete Zielbohrungen (60, 62, 64) aufweist, wobei ferner ein auf den Zielarm(10) verschiebbares, jedoch in beliebiger Position fixierbares Verbindungsteilvorgesehen ist, das eine Aufnahmeöffnung (130) für eine lösbare Befestigung eines stabartigen Nageladapters (30) hat, der seinerseits Mittel zur lösbaren Befestigung des Verriegelungsnagels (20, 43, 43a) aufweist, **dadurch gekennzeichnet, daß** der Zielabschnitt (14) des starren Zielarms (10) drei Zielbohrungen (60, 62, 64) aufweist, von denen die äußeren Zielbohrungen (60, 64) einen Abstand voneinander haben, der dem Abstand der distalen Verriegelungslöcher (22, 34; 40, 41; 40a; 41a) des Verriegelungsnagels (20, 43, 43a) entspricht und der Zielabschnitt (14) um die Achse der inneren/der äußeren Zielbohrung (60, 64) verschwenkbar gelagert ist und erste Feststellmittel (76) zur Festlegung des Zielabschnitts (14) in seiner vorgegebenen Schwenkposition.

2. System nach Anspruch 1, **dadurch gekennzeichnet, dass** der Zielabschnitt (14) im Bereich der Zielbohrungen (60 bis 64) durch einen achsparallelen Schlitz (78) senkrecht zur Achse der Zielbohrungen in einen schmaleren Abschnitt (82) und einen breiteren Abschnitt (80) geteilt ist.

3. System nach Anspruch 1 oder 2, **dadurch gekennzeichnet, dass** der Nagetadapter (30) um seine Längsachse schwenkbar im Verbindungsteil (12, 84) gelagert ist und zweite Feststellmittel (110, 114) die relative Schwenklage von Nageladapter (30) und Verbindungsteil (12, 84) zueinander festlegen.

4. System nach Anspruch 3, **dadurch gekennzeichnet, dass** die Feststellmittel zwischen dem Verbindungsteil (12, 84) und dem Nageladapter (30) vorgesehen sind zur Einstellung der relativen Schwenklage.

5. System nach einem der Ansprüche 1 bis 4, **dadurch gekennzeichnet, dass** der Zielarm (10) als Stange mit unrundem Querschnitt ausgebildet ist, die sich durch eine komplementäre Öffnung (86) des Verbindungsteils (12, 84) erstreckt, wobei eine Wand der Öffnung (86) von einem Klemmelement (88) gebildet ist, das von einer Klemmschraube (90) betätigbar ist.

6. System nach einem der Ansprüche 1 bis 5, **dadurch gekennzeichnet, dass** der Nageladapter (30) einen unrunden Schaftabschnitt (34) aufweist, der von einer komplementären Aufnahmeöffnung (130) eines Hülsenabschnitts (120) des Verbindungsteils (12, 84) verschiebbar und feststellbar aufgenommen ist, der Hülsenabschnitt (120) drehbar in einer Lagerhülse (96) des Verbindungsteils (12, 84) aufgenommen ist, deren Achse annähernd senkrecht zur Achse der anderen Öffnung (86) des Verbindungsteils (12, 84) verläuft und die zweiten Feststellmittel die relative Drehposition von Hülsenabschnitt (120) und Lagerhülse (96) fixieren.

7. System nach Anspruch 6, **dadurch gekennzeichnet, dass** mit dem ersten Hülsenabschnitt (120) ein erster Arm (122) verbunden ist, zwischen. Lagerhülse (96) und erstem Hülsenabschnitt (120) ein zweiter Hülsenabschnitt (102) angeordnet ist, der mit einem zweiten Arm (108) versehen ist, der zweite Hülsenabschnitt (102) einen dritten Arm (116) aufweist, der in verschiedenen Schwenklagen am Verbindungsteil (12, 84) fixierbar ist und zwischen dem ersten und zweiten Arm (122, 108) eine Verstellschraube angeordnet ist zur Einstellung des Abstandes der Arme (108, 122) zueinander.

8. System nach Anspruch 7, **dadurch gekennzeichnet, dass** der dritte Arm (116) einen bogenförmigen Schlitz (118) aufweist, durch den sich eine Feststellschraube (90) erstreckt, die in einer Gewindebohrung des Verbindungsteils (12,84) eingreift.

9. System nach Anspruch 8, **dadurch gekennzeichnet, dass** die Feststellschraube (90) zugleich den Zielarm (10) am Verbindungsteil (12, 84) in Längs, und Schwenkrichtung festlegt.

10. System nach Anspruch 7, **dadurch gekennzeichnet, dass** sich eine zweite Feststellschraube durch die Lagerhülse (96) und den zweiten Hülsenabschnitt (102) erstreckt und in einer Gewindebohrung des ersten Hülsenabschnitts (120) sitzt, um den Nageladapter (30) festzulegen.

11. System nach einem der Ansprüche 1 bis 10, **dadurch gekennzeichnet, dass** ein Kalibrierstift (132) vorgesehen ist mit einem Endabschnitt (134), dessen Durchmesser dem Durchmesser der Verriegelungslöcher (22, 24) entspricht, während der übrige Schaft (136) einen Durchmesser aufweist, der dem Durchmesser der Zielbohrungen (60 bis 64) entspricht.

12. System nach einem der Ansprüche 1 bis 11, **dadurch gekennzeichnet, dass** ein Fühlerstift (138) vorgesehen ist, dessen Außendurchmesser kleiner ist als der Durchmesser der Verriegelungslöcher (20, 24) und an einem Ende eine Spitze oder eine Schneide (140) aufweist.

13. System nach einem der AnspcOehe 1 bis 12, **dadurch gekennzeichnet, dass** eine längliche Fixierhülse (144) vorgesehen ist, deren Innendurchmesser dem Außendurchmesser des Fühlerstifts (138) entspricht, die einen vorderen Abschnitt (146) aufweist, dessen Außendurchmesser dem Durchmesser der Verriegelungslöcher (22, 24) entspricht und einen Abschnitt (148) aufweist, dessen Außendurchmesser dem Durchmesser der Zielbohrungen (60 bis 64) entspricht.

14. System nach einem der Ansprüche 9 bis 13, **dadurch gekennzeichnet, dass** das Verbindungsteil (84) ein Klemmelement (88) aufweist, das am Zielarm (10) angreift und einen nachgebenden Abschnitt aufweist, gegen den die Feststellschraube (90) anliegt.

15. System nach Anspruch 14, **dadurch gekennzeichnet, dass** der nachgebende Abschnitt durch einen Schlitz (92) gebildet ist.

16. System nach einem der Ansprüche 1 bis 15, **dadurch gekennzeichnet, dass** die Schwenkachse des Zielarms (10) und des Verbindungsteils (84) sich in der gleichen Ebene befinden wie die Längsachse des Verriegelungsnagels (20, 43, 43a).

## Claims

1. Implantation system comprising at least a locking nail (20, 43, 43a) and an aiming device, whereby the locking nail (20, 43, 43a) is embodied at one end for detachable attachment to the aiming device and at the other end has two locking holes (22, 24; 40, 41; 40a, 41a) and the locking nail (20, 43, 43a) has an external axially parallel groove (28) connecting the locking holes or recesses (50, 52) extending in the circumferential direction or elevations (56, 58) in the region of the locking holes and the aiming device has an aiming arm (10) with an aiming portion (14) on the free end with aiming bores (60, 62, 64) arranged in an axially parallel plane, whereby in addition a connecting piece which is displaceable on the aiming arm (10) but may be fixed in any position thereon is provided which has an accommodation aperture (130) for the detachable attachment of a rod-shaped nail adapter (30), which in turn has means for the detachable attachment of the locking nail (20, 43, 43a), **characterised in that** the aiming portion (14) of the rigid aiming arm (10) has three aiming bores (60, 62, 64), of which the external aiming bores (60, 64) have a distance from each other corresponding to the distance of the distal locking holes (22, 24; 40, 41; 40a; 41a) of the locking nail (20, 43, 43a) and the aiming portion (14) is swivel-mounted on the axis of the inner/outer aiming bore (60, 64) and first fixing means (76) for fixing the aiming portion (14) in its prespecified swivelling position.

2. System according to claim 1, **characterised in that**, in the region of the aiming bores (60 to 64), the aiming portion (14) is divided by an axially parallel slit (78) perpendicular to the axis of the aiming bores into a narrower portion (82) and a broader portion (80).

3. System according to claim 1 or 2, **characterised in that** the nail adapter (30) is swivel-mounted about its longitudinal axis in the connecting piece (12, 84) and second fixing means (110, 114) fix the relative swivelling position of the nail adapter (30) and connecting piece (12, 84) with respect to each other.

4. System according to claim 3, **characterised in that** the fixing means between the connecting piece (12, 84) and the nail adapter (30) are provided for setting the relative swivelling position.

5. System according to any one of claims 1 to 4, **characterised in that** the aiming arm (10) is formed as a bar with a noncircular cross-section which extends through a complementary aperture (86) of the connecting piece (12, 84) whereby one wall of the aperture (86) is formed by a clamping element (88) which can be actuated by a clamping screw (90).

6. System according to any one of claims 1 to 5, **characterised in that** the nail adapter (30) comprises a noncircular shaft portion (34) which is accommodated by a complementary accommodation aperture (130) of a sleeve portion (120) of the connecting piece (12, 84) in a displaceable and fixable manner (120), the sleeve portion is accommodated in a bearing sleeve (96) of the connecting piece (12, 84), the axis of which extends approximately perpendicular to the axis of the other of the other aperture (86) of the connecting piece (12, 84) and the second fixing means fix the relative rotational position of the sleeve portion (120) and bearing sleeve (96).

7. System according to claim 6, **characterised in that** a first arm (122) is connected to the first sleeve portion (120), arranged between the bearing sleeve (96) and the first sleeve portion (120) there is a second sleeve portion (102), which is provided with a second arm (108), the second sleeve portion (102) has a third arm (116) which may be fixed in different swivelling positions on the connecting piece (12, 84) and arranged between the first and second arm (122, 108), there is an adjustment screw for setting the distance of the arms (108, 122) with respect to each other.

8. System according to claim 7, **characterised in that** the third arm (116) has an arcuate slit (118) through which a fixing screw (90) extends, which engages in a threaded bore of the connecting piece (12, 84).

9. System according to claim 8, **characterised in that** the fixing screw (90) simultaneously fixes the aiming arm (10) on the connecting piece (12, 84) in the longitudinal and swivelling direction.

10. System according to claim 7, **characterised in that** a second fixing screw extends through the bearing sleeve (96) and the second sleeve portion (102) and is seated in a threaded bore of the first sleeve portion (120) in order to fix the nail adapter (30).

11. System according to any one of claims 1 to 10, **characterised in that** a calibrating pin (132) is provided with an end portion (134), the diameter of which corresponds to the diameter of the locking holes (22, 24), while the remaining shaft (136) has a diameter corresponding to the diameter of the aiming bores (60 to 64).

12. System according to any one of claims 1 to 11, **characterised in that** a feeler pin (138) is provided with an outside diameter which is small than the diameter of the locking holes (20, 24) and has a point or a cutting edge (140) on one end.

13. System according to any one of claims 1 to 12, **characterised in that** an oblong fixing sleeve (144) is provided, the inside diameter of which corresponds to the outside diameter of the feeling pin (138), which has a front portion (146) with an outside diameter corresponding to the diameter of the locking holes (22, 24) and a section (148) with an outside diameter corresponding to the diameter of the aiming bores (60 to 64).

14. System according to any one of claims 9 to 13, **characterised in that** the connecting piece (84) comprises a clamping element (88) which engages the aiming arm (10) and a resilient portion abutted by the fixing screw (90).

15. System according to claim 14, **characterised in that** the resilient portion is formed by a slit (92).

16. System according to any one of claims 1 to 15, **characterised in that** the swivelling axis of the aiming arm (10) and the connecting piece (84) are located in the same plane as the longitudinal axis of the locking nail (20, 43, 43a)

## Revendications

1. Système d'implantation constitué d'au moins un clou de verrouillage (20, 43, 43a) et d'un instrument de visée, dans lequel le clou de verrouillage (20, 43, 43a) est réalisé, à une extrémité, pour la fixation amovible à l'instrument de visée et comporte à l'autre extrémité deux trous de verrouillage (22, 24 ; 40, 41 ; 40a, 41a) et le clou de verrouillage (20, 43, 43a) comporte une rainure (28) extérieure d'axe parallèle reliant les trous de verrouillage ou des cavités (50, 52) s'étendant dans la direction périphérique ou des élévations (56, 58) dans la zone des trous de verrouillage et l'instrument de visée comporte un bras de visée (10) avec une section de visée (14) à l'extrémité libre, qui comporte des perçages de visée (60, 62, 64) disposés dans un plan d'axe parallèle, une pièce de liaison capable de coulisser sur le bras de visée (10) mais pouvant toutefois être fixée dans une position quelconque étant en outre prévue, laquelle pièce comportant un orifice de logement (130) pour une fixation amovible d'un adaptateur de clou (30) du genre tige, qui comporte pour sa part des moyens de fixation amovible du clou de verrouillage (20, 43, 43a), **caractérisé en ce que** la section de visée (14) du bras de visée (10) rigide comporte trois perçages de visée (60, 62, 64), les perçages de visée (60, 64) extérieurs ayant un écartement les uns des autres qui correspond à l'écartement des trous de verrouillage (22, 24 ; 40, 41 ; 40a, 41a) distaux du clou de verrouillage (20, 43, 43a) et **en ce que** la section de visée (14) est disposée de manière pivotante autour de l'axe du perçage de visée (60, 64) intérieur/extérieur, des premiers moyens d'immobilisation (76) étant apte à immobiliser la section de visée (14) dans sa position de pivotement donnée.

2. Système selon la revendication 1, **caractérisé en ce que**, dans la zone des perçages de visée (60 à 64), la section de visée (14) est divisée en une section plus étroite (82) et une section plus large (80) par une fente d'axe parallèle (78) perpendiculaire à l'axe des perçages de visée.

3. Système selon la revendication 1 ou 2, **caractérisé en ce que** l'adaptateur de clous (30) est disposée de manière pivotante autour de son axe longitudinal dans la pièce de liaison (12, 84), des seconds moyens d'immobilisation (110, 114) étant apte à immobiliser la position de pivotement relative de l'adaptateur de clous (30) et de la pièce de liaison (12, 84) l'un par rapport à l'autre.

4. Système selon la revendication 3, **caractérisé en ce que** les moyens d'immobilisation entre la pièce de liaison (12, 84) et l'adaptateur de clous (30) sont prévus pour régler la position de pivotement relative.

5. Système selon l'une des revendications 1 à 4, **caractérisé en ce que** le bras de visée (10) est réalisé sous forme de tige de section transversale non ronde qui s'étend à travers un orifice complémentaire (86) de la pièce de liaison (12, 84), une paroi de l'orifice (86) étant formée par un élément à pince (88) qui peut être actionné par une vis de pince (90).

6. Système selon l'une des revendications 1 à 5, **caractérisé en ce que** l'adaptateur de clous (30) présente une section de tige non ronde (34) qui est logée de manière coulissante et immobilisable par un orifice de logement (130) complémentaire d'une section de douille (120) de la pièce de liaison (12, 84), la section de douille (120) étant logée de manière capable de rotation dans une douille de support (96) de la pièce de liaison (12, 84) dont l'axe s'étend de manière approximativement perpendiculaire à l'axe de l'autre orifice (86) de la pièce de liaison (12, 84) et les seconds moyens d'immobilisation fixent la position de rotation relative de la section de douille (120) et de la douille de support (96).

7. Système selon la revendication 6, **caractérisé en ce qu'**un premier bras (122) est relié à la première section de douille (120), **en ce qu'**une deuxième section de douille (102) qui est pourvue d'un deuxième bras (108) est disposée entre la douille de support (96) et la première section de douille (120), **en ce que** la seconde section de douille (102) comporte un troisième bras (116) qui peut être fixé à la pièce de liaison (12, 84) dans différentes positions de pivotement et **en ce qu'**une vis de réglage est disposée entre le premier et le deuxième bras (122, 108) pour régler l'écartement des bras (108, 122) l'un par rapport à l'autre.

8. Système selon la revendication 7, **caractérisé en ce que** le troisième bras (116) comporte une fente en forme d'arc (118), par laquelle s'étend une vis de fixation (90) qui vient en prise dans un perçage taraudé de la pièce de liaison (12, 84).

9. Système selon la revendication 8, **caractérisé en ce que** la vis de fixation (90) immobilise le bras de visée (10) sur la pièce de liaison (12, 84), dans la direction aussi bien longitudinale que pivotante.

10. Système selon la revendication 7, **caractérisé en ce qu'**une seconde vis de fixation s'étend à travers la douille de support (96) et la seconde section de douille (102), et repose dans un perçage taraudé de la première section de douille (120), afin d'immobiliser l'adaptateur de clous (30).

11. Système selon l'une des revendications 1 à 10, **caractérisé en ce qu'**une tige de calibrage (132) est prévue avec une section d'extrémité (134) dont le diamètre correspond au diamètre des trous de verrouillage (22, 24), tandis que le reste de la tige (136) présente un diamètre qui correspond au diamètre des trous de visée (60 à 64).

12. Système selon l'une des revendications 1 à 11, **caractérisé en ce qu'**une tige de détection (138), dont le diamètre extérieur est inférieur au diamètre des trous de verrouillage (20, 24), est prévue et présente à une extrémité une pointe ou un tranchant (140).

13. Système selon l'une des revendications 1 à 12, **caractérisé en ce qu'**une douille de fixation allongée (144), dont le diamètre intérieur correspond au diamètre extérieur de la tige de détection (138) est prévue, la douille de fixation (144) comportant une section avant (146) dont le diamètre extérieur correspond au diamètre des trous de verrouillage (22, 24), est une section (148) dont le diamètre extérieur correspond au diamètre des trous de visée (60 à 64).

14. Système selon l'une des revendications 9 à 13, **caractérisé en ce que** la pièce de liaison (84), comporte un élément à pince (88) qui vient en prise sur le bras de visée (10) et comporte une section souple, contre laquelle repose la vis de fixation (90).

15. Système selon la revendication 14, **caractérisé en ce que** la section souple est formée par une fente (92).

16. Système selon l'une des revendications 1 à 15, **caractérisé en ce que** l'axe de pivotement du bras de visée (10) et de la pièce de liaison (84) se situe dans le même plan de l'axe longitudinal du clou de verrouillage (20, 43, 43a).
